Europäisches Patentamt

(19) European Patent Office

Office européen des brevets

(11) Veröffentlichungsnummer: **0 093 895**
**B1**

(12) **EUROPÄISCHE PATENTSCHRIFT**

(45) Veröffentlichungstag der Patentschrift:
07.01.87

(21) Anmeldenummer: 83103680.1

(22) Anmeldetag: 15.04.83

(51) Int. Cl.⁴: **G 01 N 3/30**, A 61 B 9/00

(54) **Perkussionsinstrument.**

(30) Priorität: 26.04.82 DE 3215530

(43) Veröffentlichungstag der Anmeldung:
16.11.83 Patentblatt 83/46

(45) Bekanntmachung des Hinweises auf die Patenterteilung:
07.01.87 Patentblatt 87/2

(84) Benannte Vertragsstaaten:
AT CH DE FR GB IT LI SE

(56) Entgegenhaltungen:
DE - A - 2 617 779
DE - A - 2 853 252
DE - A - 3 115 711
US - A - 4 289 023

(73) Patentinhaber: Siemens Aktiengesellschaft Berlin und München, Wittelsbacherplatz 2, D-8000 München 2 (DE)
Patentinhaber: Fraunhofer-Gesellschaft zur Förderung der angewandten Forschung e.V., Leonrodstrasse 54, D-8000 München 19 (DE)
Patentinhaber: Schulte, Willi, Prof. Dr., Osianderstrasse 2 - 8, D-7400 Tübingen (DE)
Patentinhaber: Lukas, Dieter, Dipl.-Phys., Osianderstrasse 2 - 8, D-7400 Tübingen (DE)

(72) Erfinder: Wohlgemuth, Jürgen, Brüder-Knauss-Strasse 61, D-6100 Darmstadt (DE)
Erfinder: Hohmann, Eugen, Am Leimenberg 32, D-6140 Bensheim (DE)
Erfinder: Bernd, Nickel, Goldregenstrasse 2, D-6143 Lorsch (DE)

(74) Vertreter: Mehl, Ernst, Dipl.-Ing. et al, Postfach 22 01 76, D-8000 München 22 (DE)

**Beschreibung**

Die Erfindung bezieht sich auf ein Perkussionsinstrument mit einem als Handstück ausgebildeten Instrumentengehäuse, in dem ein an seinem einen Ende einen Prüfkopf enthaltender Stössel aus weichmagnetischem Material beweglich gelagert ist, der einem magnetischen Antrieb unterworfen ist, welcher eine den Stössel konzentrisch umgebende Antriebsspule enthält, der aus einer Steuerelektronik aufeinanderfolgende Stromimpulse zugeführt werden, wodurch der am einen Ende des Instrumentengehäuses austretende auf eine definierte Geschwindigkeit beschleunigte und bei abgeschalteter Antriebsspule im freien Flug mit gleichbleibender Geschwindigkeit auf ein Prüfobjekt zubewegte Stössel mittels des magnetischen Antriebs wieder in seine Ausgangsposition zurückgestellt wird, und mit Einrichtungen zur Messung und Auswertung von bei Bewegungen des Stössels auftretenden Signalen.

Bei einem bekannten solchen Perkussionsinstrument (DE-A 2 617 779) wird der Stössel durch eine im vorderen, den Prüfkopf enthaltenden Teil des Instruments angeordnete und mittels einer Magnetspule in der Ausgangsstellung gehaltene Spiralfeder auf eine bestimmte Geschwindigkeit beschleunigt. Der Stössel löst sich nach vollständiger Entspannung der Feder von dieser und fliegt, in Lagern geführt, im freien Flug mit theoretisch konstanter Geschwindigkeit auf den zu prüfenden Körper zu. Nach Aufprall auf den Gegenstand wird der Stössel durch den dabei entstehenden Gegenimpuls wieder in Richtung Ausgangsstellung zurückgeworfen. Gegen Ende der Rückflugbewegung erhält die Spule wieder einen Stromimpuls. Durch das dabei entstehende Magnetfeld wird der Stössel wieder in seine Ausgangsstellung zurückgeholt und die Feder erneut gespannt. Ein mit dem Stössel über ein biegeschlaff ausgebildetes Kabel verbundener Beschleunigungsaufnehmer erfasst eine Geschwindigkeitsänderung beim Aufprall des Stössels am zu prüfenden Objekt. Diese Geschwindigkeitsänderung am zu prüfenden Objekt kann während der Auslenk- und Rückstellbewegung für bestimmte Prüf- und Diagnosezwecke in einer mit dem Beschleunigungsaufnehmer verbundenen Auswerteelektronik ausgewertet werden. In der Zahnheilkunde beispielsweise, um die Zahnbeweglichkeit bzw. den Lockerungsgrad eines Zahnes zu erfassen. Die Zeit, innerhalb der nach erfolgtem Impuls durch den Stössel der zu prüfende Zahn ausgelenkt und wieder in seine Ausgangsstellung zurückgeführt wird, gibt Aufschluss über den Zustand des Zahnhalteapparates.

Bedingt durch die Federanordnung ist insbesondere der vordere Teil des Instrumentes, an dem der Stössel mit dem Prüfkopf austritt, im Durchmesser relativ gross. Dadurch wird die Arbeit mit dem Instrument erschwert, insbesondere bei Anwendung in der zahnärztlichen Praxis. Ein weiterer wesentlicher Nachteil ist darin zu sehen, dass, wenn das Instrument in einer von der horizontalen Lage abweichenden Lage am zu prüfenden Objekt angelegt wird, die Stösselgeschwindigkeit durch die Erdbeschleunigungskraft beeinflusst wird. Diese Beeinflussung führt zu Verfälschungen der Messergebnisse. Als weiterer Nachteil hat sich gezeigt, dass die am Beschleunigungsaufnehmer befestigten und mit diesem an der Stösselbewegung teilnehmenden Anschlussleitungen bei Verdrehung des Stössels leicht reissen können. Ausserdem führen die bei dem bekannten Instrument für den Stössel vorgesehenen Verdrehsicherungen zu unerwünschten, zusätzlichen Reibungskräften.

Aus der DE-A 2 853 252 und der US-A 4 289 023 sind Perkussionseinrichtungen bekannt, bei denen sowohl der Antrieb des Stössels nach vorne, also zum Prüfobjekt hin, als auch der Rückholvorgang mittels magnetischen Antriebs erfolgt. Bei der zuletzt genannten Perkussionseinrichtung, die dazu bestimmt ist, bei Erdbohrungen die Haftverbindung zwischen einem in das Bohrloch eingesetzten Stützrohr und einem zwischen Erdbereich und Stützrohr einzubringenden Haftmittel (Zement) überprüfen zu können, ist der an beiden Enden mit kugeligem Prüfkopf ausgebildete Stössel in einem entlang der Stützrohrwandung geführten Trägerteil so geführt, dass die Prüfköpfe abwechselnd gegen die eine und die diametral gegenüberliegende Innenwandung des Stützrohres anstossen können. Die Geschwindigkeit des Stössels wird mit Hilfe einer Messeinrichtung, im dargestellten Ausführungsbeispiel in Form eines am beweglichen Stössel befestigten Permanentmagneten und einer mit diesem zusammenwirkenden Sensorspule, jeweils unmittelbar vor und nach dem Aufprall der Prüfköpfe erfasst und in einer Auswerteeinrichtung derart ausgewertet, dass man hieraus ein Mass für die Qualität der Haftverbindung ableiten, und so unter anderem ungewollte Lufteinschlüsse feststellen kann.

Abgesehen davon, dass die bekannten Perkussionseinrichtungen nicht dazu geeignet sind, als Perkussionsinstrumente mit als Handstück ausgebildetem Instrumentengehäuse vorgesehen zu werden, beschäftigen sich diese bekannten Einrichtungen auch nicht mit dem Problem der Erzielung einer stets gleichbleibenden Stösselgeschwindigkeit am Ende der Beschleunigungsphase bzw. zu Beginn der Freiflugphase.

Aufgabe der vorliegenden Erfindung ist es, ein Perkussionsinstrument der eingangs genannten Gattung dahingehend zu verbessern, dass der Stössel bei jedem Stoss stets mit der gleichen Geschwindigkeit in die Freiflugphase übergeht, so dass die Stösselgeschwindigkeit am Ende der Beschleunigungsphase auch bei Schräghaltung des Insrumentes, unbeeinflusst durch die Erdbeschleunigung, weitgehend konstant ist.

Zur Lösung der gestellten Aufgabe wird gemäss der Erfindung vorgeschlagen, dass der Stössel wenigstens zwei aneinandergrenzende Abschnitte aus einerseits nichtmagnetischem und andererseits weichmagnetischem Material enthält, von denen letzterer in der Ausgangsstellung des Stössels bis etwa zur halben Länge der Antriebsspule in diese eintaucht, dass die den Stössel konzentrisch umgebende Antriebsspule diesen auf die definierte Geschwindigkeit beschleunigt, dass eine die Grösse der Stösselgeschwindigkeit während des Beschleunigungsvorganges erfassende Messeinrichtung vorhanden ist, deren Signale einer Auswerteeinrichtung zugeführt werden, die eine Vergleicherschaltung mit Soll-Istwertvergleich für die Stösselgeschwindigkeit enthält, und dass ferner im Antriebsstromkreis Schaltmittel vorhanden sind, die von der Vergleicherschaltung aus gesteuert werden und bei Erreichen der Sollgeschwindigkeit die Stromzufuhr zur Antriebsspule abschalten.

Der Antrieb kann in bekannter Weise aus zwei Antriebsspulen bestehen; besonders vorteilhaft ist es jedoch, nur eine einzige Antriebsspule in Verbindung mit einem am Stösselende angeordneten Dauermagnetkörper vorzusehen und die Polarität der Stromimpulse für die Antriebsspule aufeinanderfolgend umzuschalten. Die Stösselgeschwindigkeit kann entweder über eine gesonderte Messspule oder über eine Integrierstufe von einem am Stössel befestigten Beschleunigungsaufnehmer gewonnen werden. In letzterem Falle kann die Messspule entfallen. Wenn, wie gemäss einer weiteren vorteilhaften Ausgestaltung der Erfindung vorgeschlagen wird, als Beschleunigungsaufnehmer ein oder mehrere plättchenförmige, in Ebenen quer zur Längsachse des Stössels angeordnete piezoelektrische Elemente, z.B. an sich bekannte piezokeramische Scheiben, vorgesehen, diese aber im vorderen, dem Prüfkopf enthaltenden Stösselabschnitt, oder im Prüfkopf selbst, angeordnet werden, lässt sich sowohl der Aufwand zur Herstellung der Beschleunigungsaufnehmer erheblich reduzieren als auch die Signalerzeugung wirkungsvoller realisieren. Denn durch die unterschiedlichen Massen, die auf die piezokeramischen Scheiben beim Aufprall und bei der Rückwärtsbewegung wirken, nämlich einerseits eine relativ grosse Masse beim Aufprall und andererseits eine demgegenüber sehr kleine Masse bei Rückführung des Stössels, können um mehrere Potenzen höhere Nutzsignale gewonnen werden als bei einer Anordnung des Beschleunigungsaufnehmers beispielsweise am anderen Stösselende. Will man auf einen Beschleunigungsaufnehmer verzichten, so können die Beschleunigungssignale auch über eine Differenzierstufe aus der Mess- und Auswerteelektronik, also in Verbindung mit der Messspule, gewonnen werden. Diese Lösung hat den Vorteil, dass durch Wegfall des Beschleunigungsaufnehmers der Stössel leichter wird und kein Anschlusskabel erforderlich ist.

Die Steuerelektronik liefert vorteilhafterweise in der Freiflugphase des Stössels einen die Lagerreibung kompensierenden Strom, indem in einer Messeinrichtung ein Geschwindigkeitsabfall gemessen und durch einen höheren Strom kompensiert wird.

Eine besonders vorteilhafte Ausgestaltung der Erfindung berücksichtigt eine unterschiedliche Instrumentenhalterung in bezug auf die Horizontale, indem die Steuerelektronik einen Grenzwertmelder enthält für eine die Neigungslage des Instruments relativ zur Waagerechten berücksichtigende maximale und minimale Stösselgeschwindigkeit in der Freiflugphase, und indem vom Grenzwertmelder bei Überschreiten der maximalen oder Unterschreiten der minimalen Stösselgeschwindigkeit aktivierte Signalgeber vorhanden sind. Extreme Schräghaltungen des Instruments, beispielsweise Abweichungen von mehr als ca. ±10° von der Horizontalen, die zu einer starken Veränderung der Stösselgeschwindigkeit in der Freiflugphase führen würden, können dem Benutzer somit für eine notwendige Korrektur der Instrumentenhaltung angezeigt werden.

Weitere vorteilhafte Ausgestaltungen und Weiterbildungen der Erfindung sind in den abhängigen Patentansprüchen enthalten.

Mehrere Ausführungsbeispiele der Erfindung werden anhand der Zeichnung näher erläutert. Es zeigen:

Fig. 1 das erfindungsgemässe Perkussionsinstrument im Längsschnitt mit den dazugehörigen Steuerbausteinen,

Fig. 2 einen Ausschnitt aus Fig. 1 zur verdrehsicheren Lagerung des Stössels,

Fig. 3 ein Blockschaltbild für das erfindungsgemässe Instrument,

Fig. 4 das Instrument in einer bestimmten Schräghaltung in bezug auf ein Prüfobjekt mit zwei dazugehörigen Diagrammen für einerseits den Antriebs- und Rückholstrom und andererseits den Geschwindigkeitsverlauf,

Fig. 5 das Instrument in einer anderen Schräghaltung als in Fig. 4 mit den enstprechenden Strom- und Geschwindigkeitsdiagrammen,

Fig. 6 eine andere Ausführungsform in schematischer Darstellung für den Aufbau des Antriebs und der Messeinrichtung für die Geschwindigkeit.

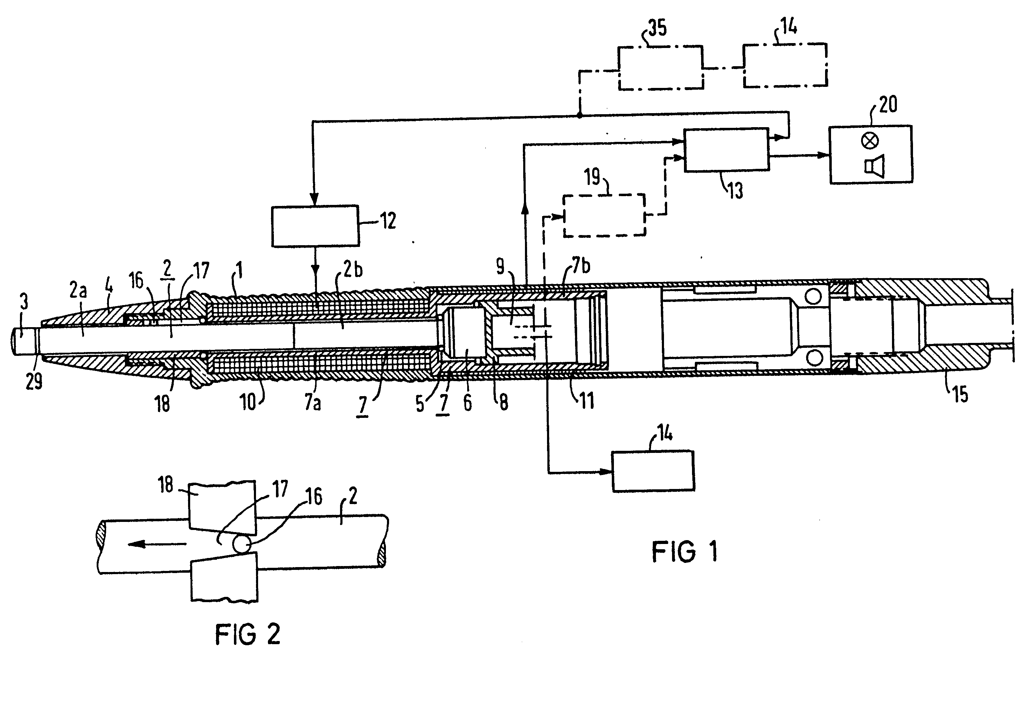

Die Fig. 1 zeigt das erfindungsgemässe Perkussionsinstrument im Längsschnitt mit der dazugehörigen Schaltung, die in dieser Figur vereinfacht und schematisiert eingetragen ist.

In einem als Handstück ausgebildeten Instrumentengehäuse 1 ist ein Stössel 2 weitgehend reibungsfrei beweglich gelagert. Der Stössel 2 enthält einen Abschnitt 2a aus nichtmagnetischem Material, z.B. Aluminium, und einen Abschnitt 2b aus weichmagnetischem Material. In der gezeichneten Position befindet sich der Stössel 2 in einer Endstellung, in der er mit seinem vorderen, den Prüfkopf 3 enthaltenden Ende aus dem konisch zulaufenden Gehäuseende 4 vorsteht. Als Anschlag dient ein O-Ring 5, der zwischen dem Stösselabschnitt 2b und einem mit diesem fest verbundenen Magnetkörper 6 an einer Schulter einer im Instrumentengehäuse 1 angeordneten Hülse 7 anliegt. Am Magnetkörper 6 fest angeordnet sind ein Flussleitstück 8 sowie ein Beschleunigungsaufnehmer 9.

Die Hülse 7 ist einstückig ausgebildet und bildet zwei konzentrisch zueinander liegende Abschnitte 7a, 7b. Um den Hülsenabschnitt 7a ist eine Antriebsspule 10 und um den Hülsenabschnitt 7b eine Messspule 11 angeordnet. Die Antriebsspule 10 ist über eine Leitung mit einer Steuerelektronik 12 verbunden, die Messspule 11 mit einer Auswerteeinrichtung 13, der Beschleunigungsaufnehmer 9 mit einer in der Darstellung schematisiert eingezeichneten Auswerteelektronik 14, in der die Beschleunigungssignale beim Auftreffen des Prüfkopfes 3 am zu prüfenden Objekt (z.B. am Zahn) abgenommen und weiterverarbeitet werden. Die erwähnten Leitungen verlaufen in üblicher Weise innerhalb des Instrumentengehäuses zu einem Anschlussteil 15, von wo aus sie zu einem ausserhalb des Instruments liegenden Unit geführt sind.

Damit der Stössel 2 ausreichend verdrehsicher geführt ist, enthält er in dem vorderen Abschnitt 2a einen quer zur Stösselachse verlaufenden Stift 16, der innerhalb des Stösselhubes in einer schwalbenschwanzförmigen Nut 17 eines im Gehäuseteil 4 gehalterten Ringes 18 geführt ist (Fig. 2). Diese Art der Verdrehsicherung ist weitgehend reibungsfrei.

Die Antriebsspule 10 ist so angeordnet, dass in der gezeichneten vorderen Endlage des Stössels 2 der Übergang vom Abschnitt 2a in den Abschnitt 2b sich etwa in der Mitte der Antriebsspule befindet. Durch die Kombination Magnetspule einerseits und Dauermagnet andererseits kann durch Umpolung der Stromimpulse durch die Antriebsspule 10 diese sowohl zur Vor- als auch zur Rückwärtsbewegung des Stössels 2 verwendet werden. Als Magnetmaterial für den Dauermagneten 6 hat sich Kobalt-Samarium als besonders vorteilhaft erwiesen; insbesondere weil aufgrund des hohen magnetischen Energieprodukts und der hohen Koerzitiv-Feldstärke dieses Magnetmaterials auf einen Eisenrückschluss um die Spule herum verzichtet werden kann. Der Baudurchmesser kann so gerade im vorderen Bereich des Instruments sehr klein gehalten werden.

Darüber hinaus trägt der Magnetkörper 6 auch zur Verstärkung der Vorwärtsbewegung des Stössels bei.

Das Flussleitstück 8 ist dazu bestimmt, den Magnetfluss durch die Messspule 11 zu richten und zu konzentrieren, so dass die durch den sich bewegenden Magneten induzierte Spannung in der Messspule 11 als Ausgangsgrösse zur Messung der Stösselgeschwindigkeit herangezogen werden kann.

Mit 20 ist eine Anzeigeeinheit bezeichnet, die akustisch oder optisch wirksam sein kann und die die aus der Messspule gewonnenen und in der Auswerteeinrichtung 13 ausgewerteten Signale für eine Anzeige verarbeitet. Bei einer unzulässigen Schräghaltung des Intruments wird so eine optische und/oder akustische Anzeige geliefert.

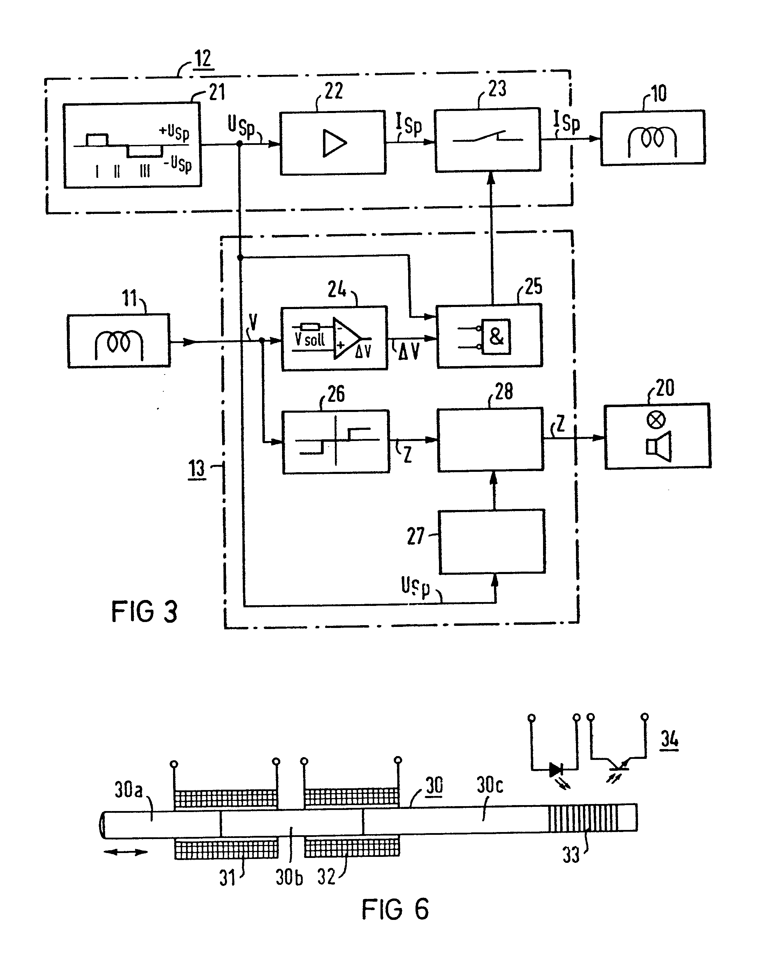

Anhand des Blockschaltbildes nach Fig. 3 wird die Wirkungsweise der Steuerelektronik sowie der Mess- und Auswerteeinrichtung 13 näher erläutert.

Die Steuerelektronik 12 enthält einen Impulsgeber 21, der für eine Vorwärtsbewegung des Stössels an die Antriebsspule 10 ein positives Signal ($+ U_{SP}$) und für die Rückwärtsbewegung ein negatives Signal ($- U_{SP}$) liefert. Zeitlich zwischen den Phasen der Vorwärtsbewegung (I) und der Rückwärtsbewegung (III) liegt die Freiflugphase (II), in der der Stössel 2 zunächst ohne zusätzlichen Antrieb nach vorne gedrückt wird. Die nachfolgende Verstärkerstufe 22 mit dem Schalter 23 schaltet entsprechend den Strom für die Antriebsspule 10. Der Schalter 23 unterbricht dabei den Antriebsstrom während der Vorwärtsbewegung (Takt I des Impulsgebers 21), wenn der Stössel 2 seine Sollgeschwindigkeit erreicht hat. Dazu wird das Geschwindigkeitssignal der Messspule 11 im Soll-Istwertvergleicher 24 mit einem vorgegebenen Sollwert verglichen. Der Schaltbefehlgeber 25 öffnet den Schalter 23, wenn die Geschwindigkeitsdifferenz ($\triangle v$) Null und die Impulsgeberspannung grösser als Null ist.

Das Geschwindigkeitssignal v wird gleichzeitig einem Grenzwertmelder 26 zugeführt, der ein Überschreiten der zulässigen Abweichungen von der Sollgeschwindigkeit während der Freiflugphase des Stössels (Takt II des Impulsgebers) misst und in diesem Fall über die Anzeigeeinheit (Signalgeber) 20 den Benutzer des Handstückes optisch oder akustisch auf eine nicht zulässige schräge Haltung des Instruments hinweist.

Der Schaltbefehlgeber 27 sorgt dafür, dass das Fehlersignal 7 vom Schalter 28 nur dann durchgeschaltet wird, wenn sich der Stössel 2 im Freiflug (Takt II des Impulsgebers) befindet. In diesem Fall ist die anliegende Spannung $U_{SP} = O$.

Bei einer Schräghaltung des Instruments wird normalerweise der Stössel zusätzlich zu der durch den Antrieb hervorgerufenen Beschleunigung auch noch durch die Erdanziehungskraft nach der Beziehung $F_E = m_{ST} \cdot g \cdot \sin \alpha$ beschleunigt, wobei $m_{ST}$ die Stösselmasse, $\alpha$ der Schräghaltungswinkel und g die Erdbeschleunigung ist.

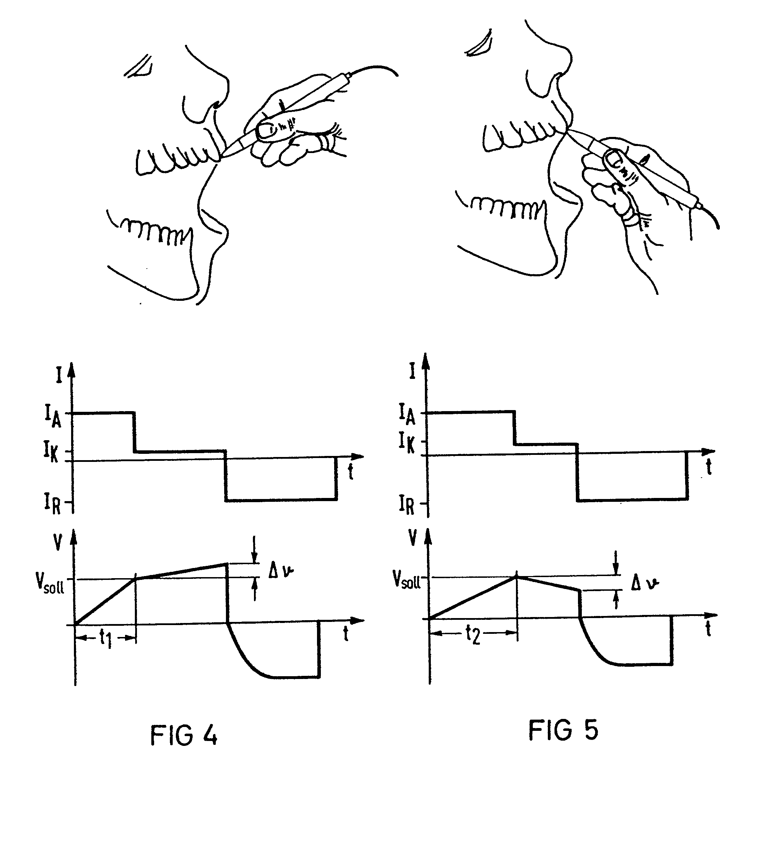

Um einen zu grossen Geschwindigkeitsfehler zu vermeiden, wird die Geschwindigkeit des Stössels, wie bereits erläutert, gemessen und mit der Sollgeschwindigkeit verglichen. Der Antriebsimpuls wird dabei genau in dem Moment abgeschaltet, in dem der Stössel seine Sollgeschwindigkeit erreicht hat. Je nachdem wie gross der Schräghaltungswinkel $\alpha$ ist, können die Antriebsimpulse verschieden lange sein. Bei sehr schräger Haltung des Instruments wird die Sollgeschwindigkeit früher erreicht sein als bei einer weniger stark geneigten Schräghaltung; demgemäss ist die Zeitdauer des Antriebsimpulses bei weniger stark geneigtem Instrument grösser als bei starker Schräghaltung. Auch während der Freiflugphase wird sich die Stösselgeschwindigkeit je nach Schräghaltung des Instruments verlangsamen bzw. erhöhen. In den Fig. 4 und 5 sind zwei unterschiedliche Positionen mit den zugehörigen Diagrammen aufgezeichnet.

Durch die in Fig. 3 aufgezeigte elektronische Schaltung wird die Abweichung der Geschwindigkeit von der Sollgeschwindigkeit erfasst und beim Überschreiten eines festgelegten zulässigen Grenzwertes während der Freiflugphase optisch oder akustisch auf diese unzulässige Fehlhaltung hingewiesen.

Ein geringfügiger Geschwindigkeitsabfall während der Freiflugphase durch Lagerreibung wird durch den Kompensationsstrom $I_K$ ausgeglichen.

Alternativ zu der in Fig. 1 aufgezeigten Lösung, bei der nur eine Antriebsspule vorhanden ist und der Rückzug durch Umpolen des Magnetflusses in Verbindung mit dem Magnetkörper (Position 6) erfolgt kann der Stösselantrieb auch durch eine Doppelspule erfolgen, wie in Fig. 6 schematisiert dargestellt ist. Der Stössel 30 besteht hier aus zwei Abschnitten 30a, 30c aus nichtmagnetischem Werkstoff und einem dazwischenliegenden Stösselabschnitt 30b aus Weicheisen. Jeweils den Übergang der beiden Abschnitte überdeckend sind im Instrumentengehäuse einerseits eine Antriebsspule 31 und eine Rückzugsspule 32 hintereinander angeordnet. Der Stössel 30 ist ferner mit einer Markierung 33 versehen, die mit einer im Instrumentengehäuse angeordneten Reflex-Lichtschranke 34 zusammenwirkt. Die Geschwindigkeit des Stössels 30 kann so optisch erfasst werden. Der konstruktive Aufbau sowie die Steuerung der Spulen erfolgen analog wie zuvor beschrieben.

Denkbar ist es, die Beschleunigungssignale nicht über einen Beschleunigungsaufnehmer zu gewinnen, sondern – wie in Fig. 1 strichpunktiert eingezeichnet – über eine Differenzierstufe 35 nach der Beziehung

$$b = \frac{dv}{dt}$$

abzuleiten. Die Differenzierstufe 35 ist an die Mess- und Auswerteeinrichtung 13 angeschlossen und wandelt die über die Messspule 11 gewonnenen Geschwindigkeitssignale in Beschleunigungssignale um, welche dann in bekannter Weise in der Auswertelektronik 14 weiterverarbeitet werden. Diese Lösung hat den Voteil, dass auf einen Beschleunigungsaufnehmer verzichtet werden kann.

Die Geschwindigkeit (v) kann auch – wie in Fig. 1 gestrichelt eingezeichnet – vom Beschleunigungsaufnehmer 9 über eine Integrierstufe 19 nach der Beziehung $v = \int b\,dt$ abgeleitet werden. In diesem Falle braucht keine Messspule vorgesehen zu werden. Die Geschwindigkeitssignale werden in der zuvor beschriebenen Weise in der Auswerteeinrichtung 13 weiterverarbeitet.

Ein besonders einfacher, insbesondere kostengünstiger Aufbau des Beschleunigungsaufnehmers mit hohem Wirkungsgrad hinsichtlich der zu gewinnenden Beschleunigungssignale lässt sich erzielen, wenn der Beschleunigungsaufnehmer nicht im hinteren, sondern im vorderen Stösselabschnitt, vorzugsweise im Prüfkopf selbst, angeordnet ist, und zwar in Form von einem oder mehreren, den Stössel in unterschiedliche Massenanteile unterteilenden Plättchen aus piezokeramischem Material.

In Fig. 1 ist eine solche Anordnung mit der Position 29 gekennzeichnet. Bei Aufprall des Stössels bzw. des Prüfkopfes auf das zu prüfende Objekt wirkt nahezu die gesamte Stösselmasse als Kraft auf die piezoelektrischen Elemente 29, mit der Folge, dass hieraus ein relativ hohes Nutzsignal gewonnen werden kann; dagegen wirkt bei der Rückführung des Stössels in die Ausgangslage und bei Anschlag an die dort vorgesehenen Begrenzungen nur eine geringe Masse, nämlich die zwischen Plättchen und Prüfkopfende, auf die piezoelektrischen Scheiben, wodurch nur ein geringfügiges Störsignal erzeugt wird.

## Patentansprüche

1. Perkussionsinstrument mit einem als Handstück ausgebildeten Instrumentengehäuse (1), in dem ein an seinem einen Ende einen Prüfkopf (3) enthaltender Stössel (2; 30) aus weichmagnetischem Material beweglich gelagert ist, der einem magnetischen Antrieb unterworfen ist, welcher eine den Stössel (2; 30) konzentrisch umgebende Antriebsspule (10; 31, 32) enthält, der aus einer Steuerelektronik (12) aufeinanderfolgende Stromimpulse ($J_A$, $J_R$) zugeführt werden, wodurch der am einen Ende des Instrumentengehäuses austretende, auf eine definierte Geschwindigkeit beschleunigte und bei abgeschalteter Antriebsspule (10; 31, 32) im freien Flug mit gleichbleibender Geschwindigkeit auf ein Prüfobjekt zubewegte Stössel mittels des magnetischen Antriebs wieder in seine Ausgangsposition zurückgestellt wird, und mit Einrichtungen zur Messung und Auswertung von bei Bewegungen des Stössels auftretenden Signalen, dadurch gekennzeichnet, dass der Stössel (2; 30) wenigstens zwei aneinandergrenzende Abschnitte (2a, 2b; 30a bis 30c) aus einerseits nichtmagnetischem und andererseits weichmagnetischem Material enthält, von denen letzterer (2b; 30b) in der Ausgangsstellung des Stössels (2; 30) bis etwa zur halben Länge der Antriebsspule (10; 31, 32) in diese eintaucht, dass die den Stössel (2; 30) konzentrisch umgebende Antriebsspule (10; 31, 32) diesen auf die definierte Geschwindigkeit beschleunigt, dass eine die Grösse der Stösselgeschwindigkeit während des Beschleunigungsvorganges erfassende Messeinrichtung (11) vorhanden ist, deren Signale einer Auswerteeinrichtung (13) zugeführt werden, die eine Vergleicherschaltung (24, 25) mit Soll-Istwertvergleich für die Stösselgeschwindigkeit enthält, und dass ferner im Antriebsstromkreis Schaltmittel (23) vorhanden sind, die von der Vergleicherschaltung (24, 25) aus gesteuert werden und bei Erreichen der Sollgeschwindigkeit die Stromzufuhr zur Antriebsspule (10; 31, 32) abschalten.

2. Perkussionsinstrument nach Anspruch 1, dadurch gekennzeichnet, dass der Stösselantrieb eine einzige Antriebsspule (10) und einen am freien Ende des weichmagnetischen Stösselabschnittes (2b) angeordneten Dauermagnetkörper (6) enthält, und dass die Steuerelektronik (12) Umschaltmittel enthält, welche die Polarität der Stromimpulse für die Antriebsspule (10) aufeinanderfolgend umschalten.

3. Perkussionsinstrument nach Anspruch 2, dadurch gekennzeichnet, dass um den Dauermagnetkörper (6) eine im Instrumentengehäuse (1) gehalterte Messspule angeordnet ist, die mit der Auswerteeinrichtung (13) für die Geschwindigkeit des Stössels (2) verbunden ist.

4. Perkussionsinstrument nach Anspruch 3, dadurch gekennzeichnet, dass die Antriebsspule (10) und die Messspule auf einem im Instrumentengehäuse (1) gehalterten einteiligen, im Durchmesser abgestuften Spulenträger (7) montiert sind.

5. Perkussionsinstrument mit einem am Stössel (2) angeordneten Beschleunigungsaufnehmer (9), der die bei jeder Stösselbewegung auftretenden Beschleunigungskräfte zur weiteren Auswertung erfasst, nach einem der Ansprüche 2 bis 4, dadurch gekennzeichnet, dass zwischen dem Beschleunigungsaufnehmer (9) und dem Dauermagnetkörper (6) ein Magnetflussleitkörper (8) angeordnet ist.

6. Perkussionsinstrument nach einem der Ansprüche 2 bis 5, dadurch gekennzeichnet, dass der Dauermagnetkörper (6) aus Kobalt-Samarium besteht.

7. Perkussionsinstrument nach Anspruch 1, mit einem am Stössel (2) angeordneten Beschleunigungsaufnehmer (9), welcher die bei jeder Stösselbewegung auftretenden Beschleunigungskräfte zur weiteren Auswertung erfasst, dadurch gekennzeichnet, dass zur Messung der Stösselgeschwindigkeit (v) eine am Beschleunigungsaufnehmer (9) angeschlossene Integrierstufe (19) vorgesehen ist.

8. Perkussionsinstrument nach Anspruch 3, dadurch gekennzeichnet, dass ein Differenzierverstärker (35) vorhanden ist, der die aus der Messspule entnehmbaren Geschwindigkeitssignale in Beschleunigungssignale umwandelt, welche in einer mit dem Differenzierverstärker (35) verbundenen Auswerteelektronik (14) ausgewertet werden.

9. Perkussionsinstrument nach Anspruch 1, dadurch gekennzeichnet, dass zur Messung der Stösselgeschwindigkeit am Stössel (2; 30) eine Markierung (33) und mit dieser korrespondierend im Instrumentengehäuse (1) eine Reflex-Lichtschranke (34) angeordnet ist.

10. Perkussionsinstrument nach einem der Ansprüche 1 bis 9, dadurch gekennzeichnet, dass der Stössel (2; 30) einen senkrecht zur Stösselachse verlaufenden Führungsstift (16) enthält, der in einer über den gesamten Verstellweg des Stössels sich erstreckenden und zum Prüfkopf (3) sich öffnenden schwalbenschwanzförmigen Nut (17) geführt ist, die an dem Gehäuseende (4), an dem der Stössel (2; 30) austritt, in einem konzentrisch um den Stössel (2; 30) angeordneten Ring (18) angeordnet ist.

11. Perkussionsinstrument nach Anspruch 1, dadurch gekennzeichnet, dass in der Freiflugphase (II) des Stössels (2; 30) die Steuerelektronik (12) einen die Lagerreibung kompensierenden Strom ($J_K$) in einer dem in der Messeinrichtung (13) gemessenen Geschwindigkeitsabfall ($\triangle$ v) entsprechenden Grösse liefert.

12. Perkussionsinstrument nach Anspruch 1, dadurch gekennzeichnet, dass die Steuerelektronik (13) einen Grenzwertmelder (26) enthält für eine die Neigungslage des Instruments relativ zur Waagerechten berücksichtigende maximale und minimale Stösselgeschwindigkeit in der Freiflugphase (II) und dass vom Grenzwertmelder (26) bei Überschreiten der maximalen Stösselgeschwindigkeit oder Unterschreiten der minimalen Stösselgeschwindigkeit aktivierte Signalgeber (20) vorhanden sind.

13. Perkussionsinstrument nach einem der Ansprüche 1 bis 12, dadurch gekennzeichnet, dass in dem den Prüfkopf (3) enthaltenden vorderen Stösselabschnitt (2a), vorzugsweise im Prüfkopf (3) selbst, ein aus einem oder mehreren plättchenförmigen, in einer Ebene/in Ebenen quer zur Längsachse des Stössels (2) angeordneten piezoelektrischen Element/Elementen bestehender Beschleunigungsaufnehmer (29) angeordnet ist.

**Claims**

1. A percussion instrument comprising an instrument casing (1) which is designed as a handpiece and wherein a plunger (2; 30), which at its one end has a testing head (3), and comprises a soft-magnetic material is movably mounted, and which plunger is subjected to a magnetic drive which comprises a driving coil (10; 31, 32) which concentrically surrounds the plunger (2; 30) and which is fed with consecutive current pulses ($J_A$, $J_R^-$) from a control electronics unit (12), whereby the plunger which emerges at the one end of the instrument casing and is accelerated to a defined speed and when the driving coil (10; 31, 32) is switched off is moved in free flight at a constant speed towards a test object, is reset into its initial position by means of the magnetic drive, and comprising devices for measuring and analysing signals which occur during movements of the plunger, characterised in that the plunger (2; 30) comprises at least two adjoining sections (2a, 2b; 30a to 30c) of a material which is non-magnetic on the one hand and soft-magnetic on the other hand, the latter of which sections (2b; 30b) enters into the driving coil for approximately half the length of said driving coil (10; 31, 32), that the driving coil (10; 31, 32) which concentrically surrounds the plunger (2; 30) accelerates the latter to the defined speed, that there is provided a measuring device (11) which measures the magnitude of the plunger speed during the accelerating process and whose signals are supplied to an analysing device (13) which comprises a comparator circuit (24, 25) with theoretical/actual value comparison for the plunger speed, and that furthermore, switching means (23) are arranged in the driving circuit, which are controlled from the comparison circuit (24, 25) and switch off the current supply to the driving coil (10; 31, 32) when the theoretical speed has been achieved.

2. A percussion instrument as claimed in claim 1, characterised in that the plunger drive comprises one single driving coil (10) and a permanent-magnet body (6) which is arranged at the exposed end of the soft-magnetic plunger section (2b), and that the control electronics unit (12) comprises change-over means which successively change over the polarity of the current pulses for the driving coil (10).

3. A percussion instrument as claimed in claim 2, characterised in that a measuring coil which is supported in the instrument casing is arranged around the permanent-magnet body (6), which measuring coil is connected to the analysing device (13) for the speed of the plunger (2).

4. A percussion instrument as claimed in claim 1, characterised in that the driving coil (10) and the measuring coil are mounted on a coil carrier (7) which is in one piece and supported in the instrument casing (1) and stepped in diameter.

5. A percussion instrument with an acceleration pick-up (9) which is arranged on the plunger (2) and which detects, for further analaysis, the acceleration forces which occur during each plunger movement, characterised in that a body (8) carrying the magnetic flux is arranged between the acceleration pick-up (9) and the permanent-magnet body (6).

6. A percussion instrument as claimed in one of claims 2 to 5, characterised in that the permanent-magnet body (6) comprises cobalt samarium.

7. A percussion instrument as claimed in claim 1, with an acceleration pick-up (9) which is arranged on the plunger (2) and detects, for further analysis, the acceleration forces which occur during each plunger movement, characterised in that in order to measure the plunger speed (v) there is provided an integrating stage (19) which is connected to the acceleration pick-up (9).

8. A percussion instrument as claimed in claim 3, characterised in that there is arranged a differentiating amplifier (35) which converts the speed signals, which can be derived from the measuring coil, into acceleration signals which are analysed in an analysing electronics unit (14) which is connected to the differentiating amplifier (35).

9. A percussion instrument as claimed in claim 1, characterised in that in order to measure the plunger speed a marker (33) is arranged on the plunger (2; 30) and a reflex light barrier (34) which corresponds to said marker is arranged in the instrument casing (1).

10. A percussion instrument as claimed in one of claims 1 to 9, characterised in that the plunger (2; 30) comprises a guide pin (16) which extends at right-angles to the plunger axis and which is guided in a dove-tailed groove (17) which extends over the overall adjusting path of the plunger and opens towards the test head (3) and which is arranged in a ring (18), which is concentrically arranged around the plunger (2; 30), at the casing end (4) at which the plunger (2; 30) emerges

11. A percussion instrument as claimed in claim 1, characterised in that in the free-flight phase (II) of the plunger (2; 30) the control electronics unit (12) supplies a current ($J_K$) which compensates for the bearing friction, with a magnitude which corresponds to the loss of speed ($\triangle$ v) measured in the measuring device (13).

12. A percussion instrument as claimed in claim 1, characterised in that the control electronics unit (13) comprises a limit value indicator (26) for a maximal and minimal plunger speed in the free-flight phase (II), which takes into consideration the inclination of the instrument relative to the horizontal, and that there are provided signal generators (20) upon overshooting the maximal plunger speed or upon undershooting the minimal plunger speed.

13. A percussion instrument as claimed in one of claims 1 to 12, characterised in that in the front plunger section (2a) which comprises the test head (3), preferably in the test head (3) itself, is arranged an acceleration pick-up (29) which comprises one or more plate-shaped piezo-electric element(s) which is/are arranged in a plane(s) transversely to the longitudinal axis of the plunger (2).

**Revendications**

1. Instrument de percussion comportant un logement (1) réalisé sous la forme d'une pièce à main et dans lequel est monté, de façon à être déplaçable, un poussoir (2; 30) constitué en un matériau magnétique doux, qui contient au niveau de l'une de ses extrémités un palpeur (3) et qui est entraîné par un dispositif d'entraînement magnétique et contient une bobine d'entraînement (10; 31, 32) entourant concentriquement le poussoir (2; 30) et à laquelle un dispositif électronique de commande (12) envoie des impulsions successives de courant ($J_A$, $J_R$), ce qui a pour effet que le poussoir, qui est ressorti au niveau d'une extrémité du logement de l'instrument, est accéléré à une vitesse définie et, lorsque la bobine d'entraînement (10; 31, 32) est débranchée, est entraîné en direction d'un objet à tester selon un déplacement libre avec une vitesse constante, est ramené dans sa position initiale au moyen du dispositif d'entraînement magnétique, et comportant des dispositifs servant à mesurer et à évaluer des signaux apparaissant lors de déplacements du poussoir, caractérisé par le fait que le poussoir (2; 30) contient au moins deux sections contiguës (2a, 2b; 30a à 30c) constituées d'une part par un matériau amagnétique et d'autre part par un matériau magnétique doux, dont la dernière (2b; 30b) pénètre dans la bobine d'entraînement (10; 31, 32) approximativement jusque sur la moitié de la longueur de cette bobine, lorsque le poussoir (2; 30) est dans sa position initiale, que la bobine d'entraînement (10; 31, 32), qui entoure concentriquement le poussoir (2; 30), accélère ce dernier pour l'amener à une vitesse définie, qu'il est prévu un dispositif de mesure (11), qui détecte la grandeur de la vitesse du poussoir pendant la phase d'accélération et dont les signaux sont envoyés à un dispositif d'évaluation (13) qui contient un circuit comparateur (24, 25) réalisant une comparaison valeur de consigne-valeur réelle pour la vitesse du poussoir, et qu'en outre il est prévu, dans le circuit d'entraînement, des moyens de commutation (23) qui sont commandés à partir du circuit comparateur (24, 25) et débranchent l'alimentation en courant envoyée à la bobine d'entraînement (10; 31, 32) lorsque la vitesse de consigne est atteinte.

2. Instrument de percussion suivant la revendication 1, caractérisé par le fait que le dispositif d'entraînement du poussoir contient une seule bobine d'entraînement (10) et un corps formant aimant permanent (6) disposé sur l'extrémité libre de la section en matériau magnétique doux (2b) du poussoir, et que le système électronique de commande (12) contient des moyens d'inversion qui inversent successivement la polarité des impulsions de courant destinées à la bobine d'entraînement (10).

3. Instrument de percussion suivant la revendication 2, caractérisé par le fait qu'autour du corps (6) formant aimant permanent se trouve disposée une bobine de mesure maintenue dans le logement (1) de l'instrument et qui est reliée au dispositif (13) d'évaluation de la vitesse du poussoir (2).

4. Instrument de percussion suivant la revendication 3, caractérisé par le fait que la bobine d'entraînement (10) et la bobine de mesure sont montées sur un porte-bobine (7) réalisé d'un seul tenant, maintenu dans le logement (1) de l'instrument et qui comporte des diamètres étagés.

5. Instrument de percussion comportant un capteur d'accélération (9) qui est monté sur le poussoir (2) et qui détecte les forces d'accélération, qui apparaissent lors de chaque déplacement du poussoir, en vue de leur évaluation ultérieure, suivant l'une des revendications 2 à 4, caractérisé par le fait qu'un corps (8) de guidage du flux magnétique est disposé entre le capteur d'accélération (9) et le corps formant aimant permanent (6).

6. Instrument de percussion suivant l'une des revendications 2 à 5, caractérisé par le fait que le corps formant aimant permanent (6) est constitué par du cobalt-samarium.

7. Instrument de percussion suivant la revendication 1, comportant un capteur d'accélération (9) qui est monté sur le poussoir (2) et qui détecte les forces d'accélération, qui apparaissent lors de chaque déplacement du poussoir, en vue de leur évaluation ultérieure, caractérisé par le fait que pour la mesure de la vitesse (v) du poussoir, il est prévu un étage intégrateur (19) raccordé au capteur d'accélération (9).

8. Instrument de percussion suivant la revendication 3, caractérisé par le fait qu'il est prévu un amplificateur différentiel (35), qui convertit les signaux de vitesse, qui peuvent être prélevés sur la bobine de mesure, en des signaux d'accélération qui sont évalués dans un système électronique d'évaluation (14) relié à l'amplificateur différentiel (35).

9. Instrument de percussion suivant la revendication 1, caractérisé par le fait que pour la mesure de la vitesse du poussoir, un repère (33) est prévu sur le poussoir (2; 30), et un relais photoélectrique à réflexion (34) est monté dans le logement (1) de l'instrument, de manière à correspondre au repère.

10. Instrument de percussion suivant l'une des revendications 1 à 9, caractérisé par le fait que le poussoir (2; 30) contient une broche de guidage (16), qui est perpendiculaire à l'axe du poussoir et est guidée dans une rainure en queue d'aronde (17), qui s'étend sur toute la trajectoire de déplacement du poussoir et s'ouvre en direction du palpeur (3) et est disposée sur l'extrémité (4) du logement, au niveau de laquelle le poussoir (2; 30) ressort, dans un anneau (18) disposé concentriquement autour du poussoir (2; 30).

11. Instrument de percussion suivant la revendication 1, caractérisé par le fait qu'au cours de la phase (II) de déplacement libre du poussoir (2; 30), le système électronique de commande (12) délivre un courant ($J_K$), qui compense le frottement des paliers et dont l'intensité correspond à la chute de vitesse ($\Delta v$) mesurée dans le dispositif de mesure (13).

12. Instrument de percussion suivant la revendication 1, caractérisé par le fait que le système électronique de commande (13) contient un transmetteur de valeurs limites (26) pour une vitesse maximale et une vitesse minimale du poussoir, tenant compte de la position inclinée de l'instrument par rapport à l'horizontale, au cours de la phase de déplacement libre (II), et qu'il est prévu des transmetteurs de signaux (20), qui sont activés par le transmetteur de valeurs limites (26) lorsque la vitesse du poussoir dépasse sa vitesse maximale ou tombe au-dessous de sa vitesse minimale.

13. Instrument de percussion suivant l'une des revendications 1 à 12, caractérisé par le fait qu'un capteur d'accélération (29), qui est constitué par un ou plusieurs éléments piézoélectriques en forme de plaquettes disposés dans un ou plusieurs plans transversalement par rapport à l'axe longitudinal du poussoir (2), est disposé dans la section avant (2a) du poussoir contenant le palpeur (3) de préférence dans ce dernier.

FIG 1

FIG 2

FIG 3

FIG 6

FIG 4

FIG 5